# EUROPÄISCHE PATENTSCHRIFT

(11) **EP 3 400 920 B1**
(45) Veröffentlichungstag und Bekanntmachung des Hinweises auf die Patenterteilung: **10.08.2022**
(21) Anmeldenummer: 17170323.4
(22) Anmeldetag: 10.05.2017
(51) Int. Cl.: A61F 15/00, A61F 13/00, A61F 13/10

(54) **VERBAND ZUR BEHANDLUNG EINER WUNDE**
DRESSING FOR TREATING A WOUND
PANSEMENT DE TRAITEMENT D'UNE PLAIE

(43) Veröffentlichungstag der Anmeldung: 14.11.2018
(73) Patentinhaber: Paul Hartmann AG, 89522 Heidenheim (DE)
(72) Erfinder: STREIT, Iris, 89522 Heidenheim (DE)

(56) Entgegenhaltungen:
- EP-A2- 0 296 325
- WO-A1-2013/055892
- WO-A2-2013/001455
- CH-A- 435 550
- DE-A1-102008 034 363
- GB-A- 2 409 977
- US-A1- 2017 105 877

## Beschreibung

Die vorliegende Erfindung betrifft einen Verband zur Behandlung einer Wunde. Der Verband umfasst eine absorbierende SAP-haltige Wundauflage und einen elastischen Schlauch zum Fixieren der Wundauflage. Weiterhin betrifft die vorliegende Erfindung eine Rolle mit einer Vielzahl von Verbänden des zuvor genannten Typs und ein Herstellungsverfahren für den zuvor genannten Verband. Die Erfindung betrifft auch eine Anordnung von Verbänden des zuvor genannten Typs.

Absorbierende Wundauflagen sind im Stand der Technik seit langem bekannt. Sie sollen die Wunde insbesondere vor schädlichen Umwelteinflüssen schützen und von der Wunde abgegebene Wundflüssigkeit aufnehmen. Für stark sezernierende Wunden werden heutzutage häufig Wundauflagen mit einem Anteil an superabsorbierenden Polymer (SAP) eingesetzt. Derartige SAP-haltige Wundauflagen können ein Vielfaches ihres Eigengewichtes an Flüssigkeit aufnehmen, wobei sich das Volumen und insbesondere das Gewicht der Wundauflagen deutlich erhöht. Diese Zunahme an Volumen und Gewicht muss bei der Befestigung von solchen Wundauflagen am Körper des Patienten berücksichtigt werden und kann problematisch sein.

SAP-haltige Wundauflagen werden zum Beispiel mit adhäsiven Filmverbänden oder Heftpflastern am Körper des Patienten befestigt. Die Filmverbände und Heftpflaster sind mit einem Klebstoff einseitig beschichtet und können an der Haut haften. Damit die SAP-haltigen Wundauflagen auch bei steigendem Volumen und steigendem Gewicht am Körper des Patienten gehalten werden können, sind die Filmverbände und Heftpflaster üblicherweise flexibel ausgestaltet und weisen eine hohe Haftkraft auf der Haut auf. Dies kann jedoch Schmerzen und Hautreizungen beim Verbandswechsel verursachen.

Es ist auch üblich SAP-haltige Wundauflagen mit Fixierbinden am Körper des Patienten zu befestigen. Dabei wird die Wundauflage mit der Fixierbinde mehrfach umwickelt. Diese Befestigungsmethode wird vor allem bei Wunden an den Extremitäten angewandt. Das Anlegen einer Fixierbinde ist jedoch zeitaufwendig. Zudem besteht die Gefahr, dass die Fixierbinde entweder zu locker oder zu fest angelegt wird. Eine zu locker angelegte Fixierbinde kann verrutschen oder sich öffnen, so dass die Wundbehandlung beeinträchtigt oder gänzlich unterbrochen wird. Eine zu fest angelegte Fixierbinde kann hingegen Schmerzen und Durchblutungsstörungen verursachen. Eine zu fest angelegte Fixierbinde ist bei SAP-haltigen Wundauflagen besonders problematisch, da sich diese während der Wundbehandlung infolge der Flüssigkeitsabsorption vergrößern können. Dadurch steigt der Druck auf die Wunde weiter an. Das korrekte Anlegen einer Binde zum Fixieren einer SAP-haltigen Wundauflage erfordert erfahrenes Pflegepersonal.

Die WO2013/055892A1 offenbart ein Kleidungsstück mit einer Wundauflage. Das Kleidungsstück hat ein Loch, um die Wundauflage aufzunehmen. Die Wundauflage weist einen Rand auf. Der Rand verbindet die Wundauflage mit dem Kleidungsstück und erstreckt sich um den Umfang des Loches, so dass die Wundauflage in dem Loch eingepasst ist. Das Einbringen des Loches in das Kleidungsstück kann jedoch einen zusätzlichen Herstellungsschritt erforderlich machen.

Der vorliegenden Erfindung liegt die Aufgabe zugrunde, einen Verband mit einer SAP-haltigen Wundauflage bereitzustellen, welcher die zuvor genannten Nachteile nicht zeigt.

Eine der vorliegenden Erfindung zugrundeliegende Aufgabe besteht somit darin, einen Verband mit einer SAP-haltigen Wundauflage bereitzustellen, welcher möglichst schmerzarm und ohne die an die Wunde angrenzende Haut zu reizen vom Körper des Patienten entfernt werden kann.

Eine weitere der vorliegenden Erfindung zugrundeliegende Aufgabe besteht darin, einen Verband mit einer SAP-haltigen Wundauflage anzugeben, der einfach und schnell am Körper des Patienten befestigt werden kann.

Eine der vorliegenden Erfindung zugrundeliegende Aufgabe ist es auch, einen Verband mit einer SAP-haltigen Wundauflage vorzuschlagen, mit dem die Wundauflage dauerhaft und ohne zu viel Druck auf die Wunde auszuüben am Patienten befestigt werden kann.

Zudem liegt der vorliegenden Erfindung die Aufgabe zugrunde, einen einfach aufgebauten und einfach herstellbaren Verband mit einer SAP-haltigen Wundauflage zur Verfügung zu stellen.

Zur Lösung dieser Aufgaben wird ein Verband nach Anspruch 1 vorgeschlagen. Der Verband umfasst eine absorbierende SAP-haltige Wundauflage. Die Wundauflage weist eine der Wunde zugewandte erste Seite und eine der Wunde abgewandte zweite Seite auf. Der Verband umfasst weiterhin einen elastischen Schlauch zum Fixieren der Wundauflage. Der Schlauch weist ein erstes offenes Ende, ein zweites offenes Ende, eine der Wunde zugewandte Innenseite und eine der Wunde abgewandte Außenseite auf. Durch die Vorsehung des elastischen Schlauches als Befestigungsmittel für die Wundauflage ist es möglich, dass der Verband ohne Einsatz von Klebstoffen am Körper des Patienten befestigt werden kann. Somit kann der Verband zum Beispiel im Rahmen eines Verbandwechsels schmerzarm und ohne die an die Wunde angrenzende Haut zu reizen vom Körper des Patienten entfernt werden. Indem der Schlauch elastisch ist, kann zudem der Druck auf die Wunde bei einer möglichen Volumenvergrößerung der Wundauflage weitestgehend konstant gehalten werden. Durch die Fixierung der Wundauflage mit dem Schlauch ist der Verband insbesondere für die Behandlung von Wunden an den Extremitäten wie Arm und Bein geeignet.

Erfindungsgemäß ist der Verband zum einen dadurch gekennzeichnet, dass die zweite Seite der Wundauflage an der Innenseite des Schlauches unlösbar befestigt ist. Die Wundauflage und der Schlauch werden also als eine Einheit bereitgestellt. Dadurch kann der Verband einfach und schnell am Körper des Patienten befestigt werden.

Zum anderen ist der erfindungsgemäße Verband dadurch gekennzeichnet, dass die zweite Seite der Wundauflage vollständig von der Innenseite des Schlauches überdeckt ist. Die Wundauflage wird also mit ihrer wundabgewandten Seite auf die Innenseite des Schlauches gelegt und an der Innenseite befestigt, wobei kein Loch oder Fenster in den Schlauch eingebracht wird, um die Wundauflage darin einzupassen. Dies ermöglicht eine besonders einfache und sichere Befestigung der Wundauflage an dem Schlauch.

Die nachfolgenden, die Wundauflage betreffenden Angaben beziehen sich insbesondere auf die Wundauflage in einem trockenen Zustand (wenn die Wundauflage also noch keine Flüssigkeit absorbiert hat).

Typischerweise ist die Wundauflage im Wesentlichen flächig ausgestaltet. Ferner weist die Wundauflage vorzugsweise eine runde oder rechteckige Form, insbesondere eine quadratische Form, auf. Flächige Wundauflagen mit den zuvor genannten Formen lassen sich leicht und sicher innerhalb des Schlauches befestigen.

Vorzugsweise umfasst die Wundauflage eine hydrophobe und im Wesentlichen flüssigkeitsundurchlässige Schicht, so dass ein Flüssigkeitsübertritt von der Wundauflage in den Schlauch im Wesentlichen verhindert werden kann. Die hydrophobe, flüssigkeitsundurchlässige Schicht ist vorteilhafterweise auch atmungsaktiv beziehungsweise wasserdampfdurchlässig. Bei der hydrophoben, flüssigkeitsundurchlässigen Schicht kann es sich zum Beispiel um eine Folie oder ein Vlies handeln, welche idealerweise auch atmungsaktiv ausgestaltet sind. Die hydrophobe, flüssigkeitsundurchlässige Schicht kann davor schützen, dass Wundflüssigkeit aus dem Verband austritt.

In einer bevorzugten Ausführungsform der Erfindung kann die Wundauflage eine die erste Seite der Wundauflage bildende Wundkontaktschicht, einen absorbierenden Kern mit dem SAP und eine die zweite Seite der Wundauflage bildende Rückschicht umfassen. Die Wundkontaktschicht ist vorzugsweise ein Vlies, insbesondere ein hydrophiles Vlies aus Polyamid und Viskose. Der absorbierende Kern kann eine Hülle aus einem hydrophilen Zellulosegewebe aufweisen, wobei sich in der Hülle hydrophile Zellulosefasern und das SAP befinden. Bei der Rückschicht kann es sich um ein hydrophobes Vlies, insbesondere ein hydrophobes Vlies aus Polypropylen, handeln. Die Rückschicht kann die zuvor genannte hydrophobe, flüssigkeitsundurchlässige Schicht darstellen, welche einen Flüssigkeitsübertritt von der Wundauflage in den Schlauch verhindert. Eine Wundauflage mit einem solchen Aufbau kann viel Wundflüssigkeit aufnehmen und wird von der Anmelderin unter der Bezeichnung Zetuvit Plus^{®} vertrieben.

Das in der Wundauflage enthaltene superabsorbierende Polymer (SAP) ist normalerweise ein Polyacrylat. Zudem liegt das SAP vorzugsweise in Partikelform vor. SAP auf Basis von Polyacrylat besitzt eine hohe Wasseraufnahmefähigkeit.

Der erfindungsgemäße Verband ist insbesondere zur Befestigung von SAP-haltigen Wundauflagen mit einer hohen Saugkraft vorgesehen. Eine solche Wundauflage kann zum Beispiel eine Saugleistung von mindestens 50g/100cm², mindestens 100g/100cm² oder mindestens 140g/100cm², gemessen nach DIN EN 13726-1 (Kapitel 3.2), aufweisen. Die angegebenen Werte können sich auch auf den absorbierenden Kern der Wundauflage beziehen, falls ein solcher vorhanden ist.

Weiterhin ist es vorteilhaft, wenn die erste Seite der Wundauflage mit Silikon beschichtet ist. Damit kann ein Verkleben der Wundauflage mit der Wunde vermieden werden. Entsprechend kann auch die zuvor erwähnte Wundkontaktschicht silikonbeschichtet sein.

Vorteilhaft ist auch, wenn die Wundauflage Aktivkohle, insbesondere eine Schicht mit Aktivkohle, umfasst, um von der Wundflüssigkeit ausgehende unangenehme Gerüche zu neutralisieren.

Schläuche zum Fixieren von Wundauflagen (Schlauchverbände) sind prinzipiell im Stand der Technik bekannt und im Handel leicht verfügbar, wie zum Beispiel das Produkt Coverflex^{®} der Anmelderin. Die bekannten Schläuche sind wie der Schlauch des erfindungsgemäßen Verbandes im Allgemeinen beweglich und elastisch ausgestaltet.

Vorzugsweise besitzt der Schlauch im ungedehnten Zustand einen im Wesentlichen konstanten Innendurchmesser. Dies erleichtert die Befestigung der Wundauflage an dem Schlauch.

Der Schlauch im ungedehnten Zustand kann als die Mantelfläche eines geraden Kreiszylinders aufgefasst werden. Der Schlauch im ungedehnten Zustand kann also in die Form einer Mantelfläche eines geraden Kreiszylinders gebracht werden. Dies ist eine typische Form für Schlauchverbände.

In einer besonders bevorzugten Ausführungsform der Erfindung ist der Schlauch im Wesentlichen flüssigkeitsundurchlässig ausgestaltet. Hierfür kann der Schlauch hydrophob ausgebildet sein. Vorzugsweise ist der Schlauch darüber hinaus atmungsaktiv, also wasserdampfdurchlässig. Der Schlauch kann ein elastisches Gewebe, ein elastisches Gewirke und/oder ein elastisches Vlies umfassen. Die Elastizität des Vlieses kann durch in das Vlies eingearbeitete Elastikfäden erzielt werden. Insbesondere umfasst der Schlauch ein elastisches Vlies aus Polypropylen beziehungsweise ein Vlies aus Polypropylen mit Elastikfäden. Die genannten Elastikfäden können aus Polyurethan sein. Ein elastisches Vlies aus Polypropylen kann flüssigkeitsundurchlässig und atmungsaktiv sein. Ein flüssigkeitsundurchlässiger Schlauch hat den Vorteil, dass Wundflüssigkeit nicht aus dem Verband austreten kann. Der Austritt von Wundflüssigkeit aus dem Verband kann besonders effektiv verhindert werden, wenn die Wundauflage die zuvor erwähnte hydrophobe, flüssigkeitsundurchlässige Schicht umfasst und zusätzlich der Schlauch flüssigkeitsundurchlässig ist.

Es kann auch von Vorteil sein, wenn der Schlauch nur aus einem der zuvor genannten Materialien besteht. Entsprechend kann der Schlauch aus einem einzigen elastischen Gewebe, einem einzigen elastischen Gewirke oder einem einzigen elastischen Vlies bestehen. Ein Schlauch aus einem einzigen Material ist einfacher und preisgünstiger in der Herstellung. Die nachfolgend beschriebenen Bereiche unterschiedlicher Elastizität sowie der Silikonbund sind in dieser sehr einfachen Ausgestaltung des Schlauches nicht vorgesehen.

Denkbar ist auch, dass der Schlauch Bereiche unterschiedlicher Elastizität aufweist. Zum Beispiel könnte ein die Wundauflage überdeckender Bereich des Schlauches eine geringere Elastizität als ein nicht die Wundauflage überdeckender Bereich des Schlauches aufweisen. Damit könnte die Verbindung von Wundauflage und Schlauch verbessert werden, da das Dehnen des Schlauches gegebenenfalls die Verbindungsmittel zwischen Wundauflage und Schlauch schwächt.

Üblicherweise ist der Schlauch nicht-adhäsiv ausgebildet. Das bedeutet, dass der Schlauch keinen mit dem Körper des Patienten in Berührung kommenden Klebstoff umfasst, um die Wundauflage an dem Körper des Patienten zu befestigen. Für die Befestigung der Wundauflage am Körper des Patienten wird in erster Linie die Elastizität des Schlauches genutzt. Dennoch kann der Schlauch unterstützend mindestens einen Silikonbund aufweisen. Der Silikonbund befindet sich im Allgemeinen an oder nahe einer Schlauchöffnung. Die Haftkraft des Silikonbundes auf der Haut ist sehr gering, kann aber ein mögliches Verrutschen des Verbandes verhindern. Der Silikonbund kann zum Beispiel einen streifenartigen Bereich der Schlauchinnenseite umfassen, wobei die Schlauchinnenseite in dem streifenartigen Bereich silikonisiert ist. Ein solcher Silikonbund beziehungsweise eine solche Silikonisierung wird vorliegend nicht als Klebstoff (Adhäsiv) aufgefasst.

Vorstellbar wäre es auch, nicht nur einen Silikonbund vorzusehen, sondern die gesamte, nicht mit der Wundauflage in Kontakt stehende Schlauchinnenseite zu silikonisieren. Damit könnte ein mögliches Verrutschen des Verbandes besonders effektiv verhindern werden. Damit der Schlauch trotz silikonisierter Schlauchinnenseite noch elastisch und atmungsaktiv ist, könnten Öffnungen beziehungsweise Perforationen in der Silikonschicht vorhanden sein. Zum Beispiel könnte die Silikonisierung der Schlauchinnenseite in einem punktförmigen Muster erfolgen.

Die Abnahme des Verbandes kann weiter vereinfacht werden, wenn der Schlauch eine Sollriss-Stelle aufweist. Der Verband muss dann nicht aufgedehnt und von dem zu behandelnden Körperteil, zum Beispiel einem Arm, gezogen werden, sondern kann entlang der Sollriss-Stelle geöffnet und wie eine herkömmliche flächige Wundauflage von der Körperoberfläche abgenommen werden. Es besteht dann auch eine geringere Gefahr, dass die gegebenenfalls infektiöse Wundflüssigkeit enthaltene Wundauflage mit intakter Haut des Patienten in Berührung kommt. Die Sollriss-Stelle kann eine perforierte Linie sein, die über einen Teil oder die gesamte Länge des Schlauches verläuft.

Wie bereits erwähnt, ist die zweite Seite der Wundauflage an der Innenseite des Schlauches unlösbar befestigt. Dabei kann ein Teilbereich der zweiten Seite der Wundauflage oder die gesamte zweite Seite der Wundauflage an der Innenseite des Schlauches unlösbar befestigt sein. Letzteres führt zu einer besonders festen und dauerhaften Verbindung von Wundauflage und Schlauch.

Die Wundauflage und der Schlauch können zum Beispiel miteinander vernäht werden, um die Verbindung zwischen Wundauflage und Schlauch herzustellen. Dabei kann es vorteilhaft sein, einen elastischen Faden einzusetzen, damit die Nähte bei Dehnung des Verbandes nicht reißen können. Alternativ kann die zweite Seite der Wundauflage an der Innenseite des Schlauches mit einem Klebstoff, insbesondere mit einem Schmelzklebstoff, oder durch Ultraschallschweißen unlösbar befestigt sein. Mit einem Klebstoff kann die zuvor genannte vollflächige Befestigung der Wundauflage an dem Schlauch am einfachsten erzielt werden.

Da der Schlauch die Wundauflage aufnehmen und sicher fixieren soll, ist der Schlauch normalerweise größer als die Wundauflage. Entsprechend ist eine von der Innenseite des Schlauches gebildete Fläche A_{SL} normalerweise größer als eine von der zweiten Seite der Wundauflage gebildete Fläche A_{WA}. Üblicherweise ist die Fläche der Schlauchinnenseite (A_{SL}) mindestens um den Faktor 2 größer als die Fläche der wundabgewandten Seite der Wundauflage (A_{WA}). Insbesondere ist die Fläche der Schlauchinnenseite (A_{SL}) mindestens um den Faktor 3, 4, 5 oder 6 größer als die Fläche der wundabgewandten Seite der Wundauflage (A_{WA}). Umso größer der freie, nicht mit der Wundauflage in Kontakt stehende Bereich des Schlauches ist (umso größer also der zuvor genannte Faktor ist), umso weniger kann die Elastizität des Schlauches von einer nicht-elastischen Wundauflage beeinträchtigt werden. Meistens wird der zuvor genannte Faktor jedoch den Wert von 10 nicht überschreiten.

Normalerweise ist die Wundauflage zudem auf eine Hälfte des Schlauches in Längsrichtung begrenzt. Andernfalls könnte eine nicht-elastische Wundauflage den Schlauch zu sehr versteifen, wenn die Wundauflage und der Schlauch wie erfindungsgemäß gefordert miteinander verbunden werden. Entsprechend ist die Wundauflage normalerweise in einer ersten Schlauchhälfte und nicht in einer zweiten Schlauchhälfte, bezogen auf eine Schnittebene entlang einer Längsachse des Schlauches, angeordnet. Die Längsachse verläuft innerhalb des Schlauches. Die erste und die zweite Schlauchhälfte ergeben den Schlauch und sind gleich groß.

Weiterhin können die Größen von Wundauflage und Schlauch so aufeinander abgestimmt sein, dass die Wundauflage an das erste und das zweite offene Ende angrenzt. Wundauflage und Schlauch weisen dann die gleiche Länge auf. Ein solcher Verband ist für großflächige Wunden abseits von Gelenken geeignet. Wundauflage und Schlauch können in ihrer Größe jedoch auch so aufeinander abgestimmt sein, dass die Wundauflage von dem ersten und dem zweiten offenen Ende beabstandet ist. Die Länge der Wundauflage ist dann kleiner als die Länge des Schlauches. Ein solcher Verband ist für Wunden an Gelenken geeignet und kann die Wundauflage besonders gut fixieren. Zum Beispiel kann der Abstand der Wundauflage von dem ersten und dem zweiten offenen Ende jeweils mindestens etwa 2 cm, vorzugsweise mindestens etwa 4 cm oder mindestens etwa 6 cm, betragen. Die Wundauflage ist dann mindestens etwa 2 cm, 4 cm oder 6 cm von sowohl dem ersten als auch dem zweiten offenen Ende beabstandet. Größere Abstände der Wundauflage zu den Enden begünstigen im Allgemeinen die fixierende Wirkung des Schlauches. In der Regel ist die Wundauflage jedoch nicht mehr als circa 15 cm von sowohl dem ersten als auch dem zweiten offenen Ende beabstandet.

Falls der Schlauch aus einem undurchsichtigen Material besteht, kann die unter dem Schlauch liegende Wundauflage gegebenenfalls nicht mehr bei Anblick der Schlauchaußenseite gesehen werden. Es ist jedoch von Vorteil, wenn ein die Wundauflage überdeckender Bereich des Schlauches (unter dem die Wundauflage positioniert ist) an der Außenseite visuell erkennbar ist. Zum Beispiel kann der die Wundauflage überdeckende Bereich des Schlauches an der Außenseite durch eine Umrisszeichnung und/oder durch eine farbige Markierung visuell erkennbar sein. Dabei entsprechen die Umrisszeichnung und die farbige Markierung in Position, Größe und Form im Wesentlichen der zweiten Seite der Wundauflage. Die Position der Wundauflage unter dem Schlauch ist dann besser identifizierbar und ein exaktes Platzieren der Wundauflage auf der Wunde ist erleichtert. Ebenso können die Umrisszeichnung und die farbige Markierung ein Verrutschen der Wundauflage nach dem Anlegen des Verbandes besser erkennen lassen.

Die derzeit im Handel verfügbaren SAP-haltigen Wundauflagen sind zwar biegsam und weich, um sich unterschiedlichen Wundkonturen anpassen zu können, jedoch nicht zwangsläufig auch elastisch. Im Zusammenhang mit der vorliegenden Erfindung kann es allerdings vorteilhaft sein, wenn nicht nur der Schlauch, sondern auch die Wundauflage elastisch ausgebildet ist. Beispielsweise könnten Wundauflage und Schlauch in etwa die gleiche Elastizität besitzen. Damit kann das Anlegen des Verbandes am Patienten weiter vereinfacht werden.

Gegenstand der vorliegenden Erfindung ist auch eine Rolle umfassend eine Vielzahl von Verbänden nach Anspruch 1 (und den bevorzugten Ausführungsformen des Verbandes nach Anspruch 1), wobei die Verbände trennbar miteinander verbunden sind. Die Bereitstellung der Verbände auf einer Rolle ist platzsparend und ermöglicht eine Anpassung der Verbandslänge. Die Verbände können von einer Rolle auch leichter entnommen werden als beispielsweise von einem Stapel.

Vorzugsweise sind die Verbände mit Sollriss-Stellen lösbar miteinander verbunden. Diese Sollriss-Stellen verlaufen quer zur Längsachse der Verbände und sind typischerweise perforierte Linien. Werden zwei angrenzende Verbände an der Sollriss-Stelle voneinander getrennt, entsteht eine der anspruchsgemäßen Öffnungen an den beiden Verbänden. Eine besonders gute Längenanpassung kann erzielt werden, wenn die Wundauflagen der aufgerollten Verbände eine durchgehende Reihe oder Bahn bilden. Dies kann erreicht werden, wenn bei jedem Einzelverband der Rolle die Wundauflage an das erste und das zweite offene Ende des Schlauches (beziehungsweise die Sollriss-Stellen) angrenzt.

Falls keine Sollriss-Stellen vorhanden sind, können die aufgerollten Verbände zum Beispiel durch Abschneiden voneinander getrennt werden, wobei die anspruchsgemäßen Schlauchöffnungen entstehen. In diesem Fall ist es jedoch vorteilhaft, wenn zwischen den Wundauflagen der Einzelverbände Lücken vorhanden sind. In den Lücken sollen die Schnitte erfolgen, damit die Wundauflagen nicht beschädigt werden können. Vorzugsweise umfasst die Rolle Indizierungen, zum Beispiel in Form von Linien, um anzuzeigen, an welchen Stellen die Rolle geschnitten werden kann ohne die Wundauflage anzuschneiden und damit gegebenenfalls zu beschädigen. Die zuvor erwähnte Umrisszeichnung oder farbige Markierung kann gleichfalls dabei helfen, eine Beschädigung der Wundauflagen beim Zerschneiden der Rolle zu verhindern.

Um die Handhabung und die Lagerung der Rolle zu verbessern, kann die Rolle weiterhin eine Spule umfassen und/oder in einer Spenderbox untergebracht sein. Die Spule kann zum Beispiel das Aufrollen der Verbände zu der Rolle vereinfachen und der Rolle Stabilität verleihen. Die Spule könnte in der Art einer Kabelspule ausgebildet sein, die an einer Halterung drehbar befestigt ist. Die Rolle kann auch in einer Spenderbox untergebracht sein, um sie insbesondere während der Lagerung vor schädlichen äußeren Einflüssen zu schützen. Derartige Spenderboxen für Produkte zur Wundbehandlung sind im Stand der Technik prinzipiell bekannt, zum Beispiel aus der DE1891083U. Denkbar ist auch eine Rolle mit einer Spule in Kombination mit einer Spenderbox, wobei die Spule eine rotierende Achse in der Spenderbox ausbildet, um das Abrollen der Verbände zu erleichtern. Zudem kann die Spenderbox eine Abrisskante zum Abtrennen eines Verbandes von der Rolle umfassen. Typischerweise umfasst die Abrisskante eine Schneide oder ein sägeblattartiges Element. Die zusätzliche Abrisskante ist insbesondere dann von Vorteil, wenn die Rolle zum Abtrennen eines Verbandes zerschnitten werden soll. Die Abrisskante erleichtert das Zerschneiden der Rolle, weil das zum Zerschneiden der Rolle benötigte Werkzeug in die Spenderbox integriert ist und nicht separat bereitgestellt, vorrätig gehalten und bedient werden muss.

Ein weiterer Gegenstand der vorliegenden Erfindung ist eine Anordnung von Verbänden zur Behandlung von Wunden an unterschiedlichen Körperstellen, umfassend
- einen oder mehrere erste Verbände nach Anspruch 1 (und den bevorzugten Ausführungsformen des Verbandes nach Anspruch 1), wobei die Wundauflage eines jeden ersten Verbandes an das jeweilige erste und zweite offene Ende angrenzt, und
- einen oder mehrere zweite Verbände nach Anspruch 1 (und den bevorzugten Ausführungsformen des Verbandes nach Anspruch 1), wobei die Wundauflage eines jeden zweiten Verbandes von dem jeweiligen ersten und zweiten offenen Ende beabstandet ist.

Der Abstand der Wundauflage eines jeden zweiten Verbandes von dem jeweiligen ersten und zweiten offenen Ende beträgt vorzugsweise jeweils mindestens etwa 2 cm, 4 cm oder 6 cm. In der Regel sind die Wundauflagen der zweiten Verbände jedoch nicht mehr als circa 15 cm von sowohl deren ersten als auch deren zweiten offenen Ende beabstandet.

Mit der Anordnung können in vorteilhafter Weise Verbände für die Behandlung von Wunden an unterschiedlichen Körperstellen bereitgestellt werden. Die ersten Verbände sind für die Behandlung von Wunden abseits von Gelenken angepasst. Insbesondere sind die ersten Verbände für die Behandlung von großflächigen Wunden abseits von Gelenken angepasst. Das heißt, die ersten Verbände sind dafür vorgesehen, an einem Körperteil ohne Gelenk befestigt zu werden (wie zum Beispiel einem Unterarm oder Unterschenkel). Die zweiten Verbände sind für die Behandlung von Wunden an Gelenken angepasst. Insbesondere sind die zweiten Verbände für die Behandlung von kleineren und schwer zugänglichen Wunden an Gelenken angepasst. Zum Beispiel können Wunden am Ellbogen, am Knie oder auch an der Ferse in vorteilhafter Weise mit den zweiten Verbänden behandelt werden.

Ein letzter Gegenstand der vorliegenden Erfindung ist ein Verfahren zur Herstellung eines Verbandes für die Behandlung einer Wunde nach Anspruch 1 (und den bevorzugten Ausführungsformen des Verbandes nach Anspruch 1), umfassend die Schritte:
i. Bereitstellen einer SAP-haltigen Wundauflage, wobei die Wundauflage eine der Wunde zugewandte erste Seite und eine der Wunde abgewandte zweite Seite aufweist,
ii. Bereitstellen eines elastischen Schlauches zum Fixieren der Wundauflage, wobei der Schlauch ein erstes offenes Ende, ein zweites offenes Ende, eine der Wunde zugewandte Innenseite und eine der Wunde abgewandte Außenseite aufweist,
iii. Befestigen der zweiten Seite der Wundauflage an der Innenseite des Schlauches, so dass Wundauflage und Schlauch unlösbar miteinander verbunden sind und die zweite Seite der Wundauflage vollständig von der Innenseite des Schlauches überdeckt ist.

Die vorangehend beschriebenen zusätzlichen Merkmale des Verbandes (bevorzugte Ausführungsformen) sind auf die Verbände der Rolle, der Anordnung und den mit dem Verfahren hergestellten Verband übertragbar.

### Figuren

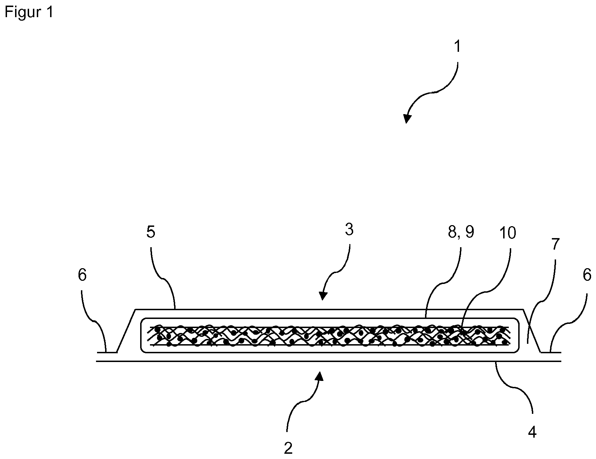

Der mit dem vorliegenden Dokument vorgeschlagene Verband wird im Folgenden anhand der **Figuren 1 bis 9** schematisch und beispielhaft veranschaulicht. Dabei können identische Strukturelemente mit denselben Bezugszeichen ausgewiesen sein.

**Figur 1** zeigt einen Querschnitt einer absorbierenden SAP-haltigen Wundauflage **1** in einer bevorzugten Ausführungsform der Erfindung. Die in **Figur 1** gezeigte Wundauflage **1** kann ein Bestandteil des erfindungsgemäßen Verbandes sein. Die Wundauflage **1** weist eine der Wunde (nicht dargestellt) zugewandte erste Seite **2** und eine der Wunde abgewandte zweite Seite **3** auf. Die erste Seite **2** wird von einer Wundkontaktschicht **4** der Wundauflage **1** gebildet, während die zweite Seite **3** von einer Rückschicht **5** der Wundauflage **1** gebildet wird. Bei der Wundkontaktschicht **4** kann es sich zum Beispiel um ein hydrophiles Vlies aus Polyamid und Viskose in einem Mischungsverhältnis von 55:45 handeln. Bei der Rückschicht **5** kann es sich um ein hydrophobes Vlies aus Polypropylen handeln. Die Wundauflage **1** ist flächig und vorzugsweise quadratisch ausgebildet und kann beispielsweise eine Abmessung von circa 10cm × 10cm besitzen. Die Wundkontaktschicht **4** und die Rückschicht **5** sind in einem Randbereich **6** der Wundauflage **1** miteinander verbunden und bilden eine Tasche **7** aus. In der Tasche **7** ist ein absorbierender Kern **8** mit dem SAP angeordnet. Der Kern **8** hat eine Hülle **9**, innerhalb der sich das SAP befindet. Vorzugsweise handelt es sich bei der Hülle **9** um ein hydrophiles Zellulosegewebe, in welchem sich eine Mischung **10** von hydrophilen Zellulosefasern und dem SAP befindet ("Fluff").

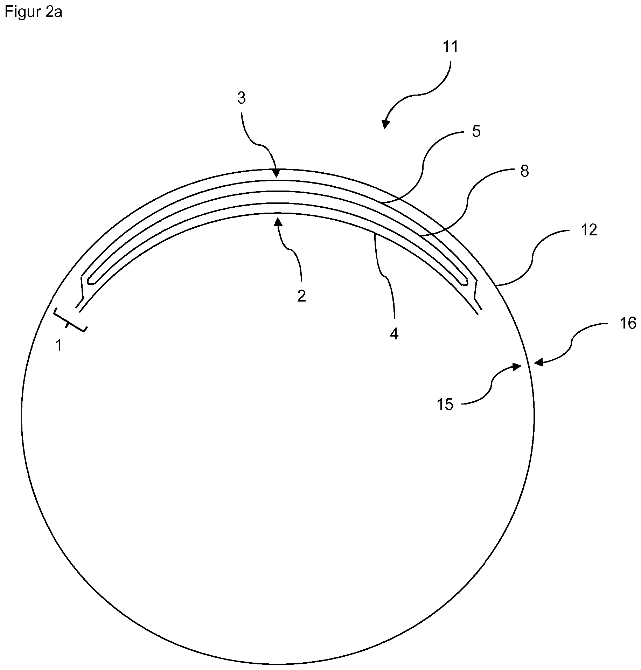

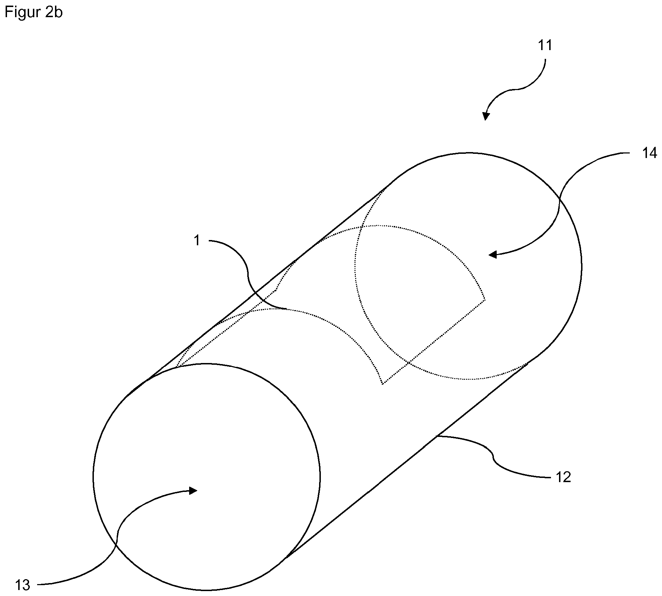

Die **Figuren 2a** **und** **2b** zeigen einen Querschnitt und eine perspektivische Ansicht eines Verbandes **11** gemäß einer ersten Ausführungsform der Erfindung. Der Verband **11** umfasst die Wundauflage **1** aus **Figur 1**. Weiterhin umfasst der Verband einen elastischen Schlauch **12** zum Fixieren der Wundauflage **1** am Patienten. Der Schlauch **12** weist wie aus **Figur 2b** hervorgeht ein erstes offenes Ende **13** und ein zweites offenes Ende **14** auf, damit das zu behandelnde Körperteil (zum Beispiel ein Arm- oder Beinabschnitt) in den Verband eingebracht werden kann. Der Schlauch **12** weist zudem eine der Wunde (nicht dargestellt) zugewandte Innenseite **15** und eine der Wunde abgewandte Außenseite **16** auf (siehe **Figur 2a****).** Der Schlauch **12** hat in dem dargestellten ungedehnten Zustand einen im Wesentlichen konstanten Innendurchmesser und umfasst vorzugsweise ein elastisches Vlies aus Polypropylen. Wesentlich an dem Verband **11** ist, dass die zweite Seite **3** der Wundauflage **1** an der Innenseite **15** des Schlauches **12** unlösbar befestigt ist. Die Befestigung kann beispielsweise mit einem Schmelzklebstoff (nicht dargestellt) erfolgen. Die Befestigung der Wundauflage **1** an dem Schlauch **12** erfolgt derart, dass die zweite Seite **3** der Wundauflage **1** vollständig von der Innenseite **15** des Schlauches **12** überdeckt ist. Die Überdeckung der Wundauflage **1** von dem Schlauch **12** soll in **Figur 2b** mit der gestrichelten Darstellung der Wundauflage **1** zum Ausdruck gebracht werden.

Wie insbesondere aus **Figur 2b** hervorgeht, ist die Wundauflage **1** deutlich kleiner als der Schlauch **12** und in einem zentralen Abschnitt des Schlauches **12** angeordnet. Somit ist die Wundauflage **1** von den offenen Enden **13**, **14** beabstandet.

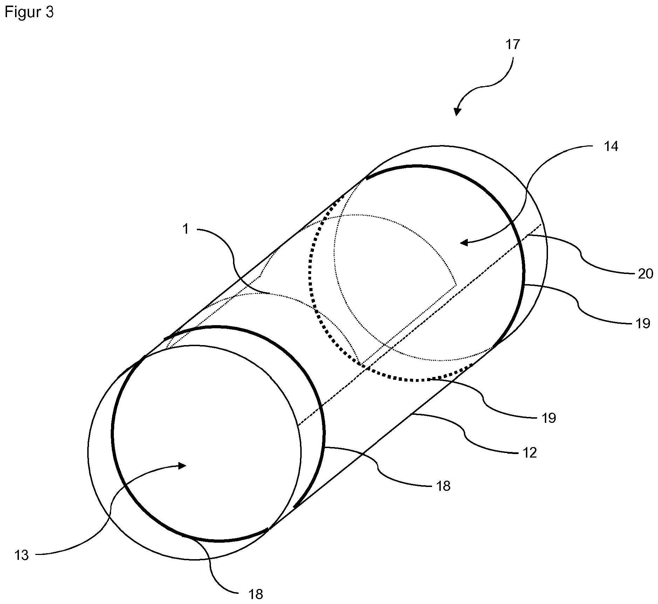

**Figur 3** zeigt eine perspektivische Ansicht eines Verbandes **17** gemäß einer zweiten Ausführungsform der Erfindung. Der Verband **17** ist wie der Verband **11** aufgebaut, hat jedoch die folgenden zusätzlichen Merkmale. Um ein Verrutschen des Verbandes **17** am Körper des Patienten zu verhindern, sind nahe der Öffnungen **13**, **14** jeweils ein Silikonbund **18**, **19** angebracht. Ein weiterer Vorteil des Verbandes **17** gegenüber dem Verband **11** ergibt sich aus der als Perforation ausgebildeten Sollriss-Stelle **20**, welche die Abnahme des Verbandes **17** bei einem Verbandswechsel vereinfacht.

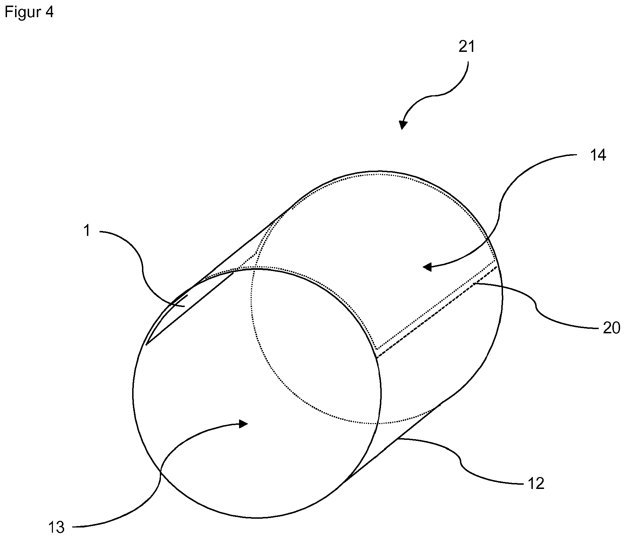

**Figur 4** zeigt eine perspektivische Ansicht eines Verbandes **21** gemäß einer dritten Ausführungsform der Erfindung. Im Unterschied zu den vorangehend beschriebenen Verbänden **11**, **17** grenzt bei Verband **21** die Wundauflage **1** an die offenen Enden **13**, **14** an. Außerdem hat der Verband **21** aufgrund fehlender freier Schlauchüberstände keinen Silikonbund.

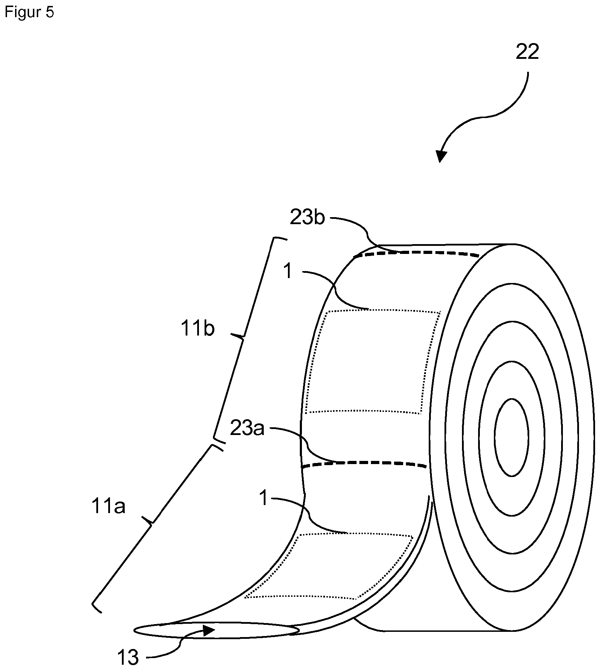

**Figur 5** zeigt eine Rolle **22** in einer ersten Ausführungsform der Erfindung. Die Rolle **22** umfasst eine Vielzahl von Verbänden nach **Figur 2**. Am Ende der Rolle **22** ist ein erster Verband **11a** und ein zweiter Verband **11b** zu erkennen. Die Einzelverbände der Rolle **22** sind trennbar miteinander verbunden. Hierfür sind Sollriss-Stellen in Form von quer zur Längsachse der Verbände verlaufender Perforationen in der Rolle **22** eingebracht. In **Figur 5** sind zwei dieser Perforationen mit den Bezugszeichen **23a** und **23b** ausgewiesen. Wird der Verband **11a** von der Rolle **22** entlang der Perforation **23a** gelöst, so entsteht die zweite Öffnung des Verbandes **11a** und die erste Öffnung des Verbandes **11b**.

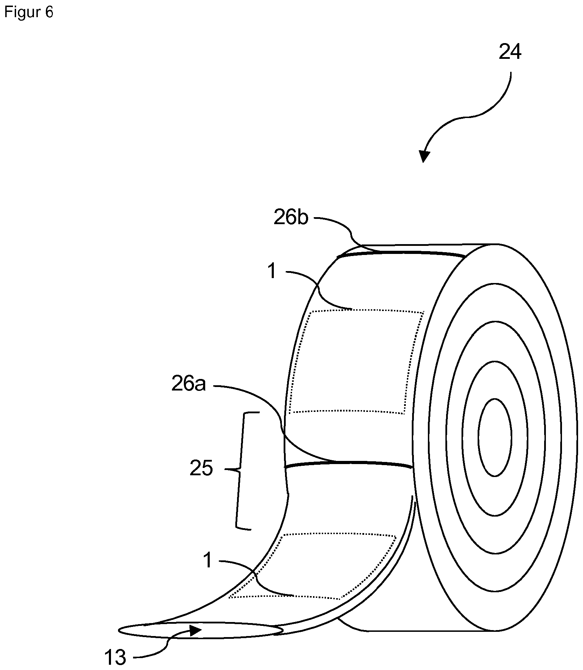

**Figur 6** zeigt eine Rolle **24** in einer zweiten Ausführungsform der Erfindung. Die Rolle **24** ist ganz ähnlich wie die Rolle **22** aus **Figur 5** ausgestaltet. Die Rolle **24** hat jedoch keine Perforationen. Die Einzelverbände werden von der Rolle **24** durch Abschneiden gelöst. Damit die Wundauflagen **1** beim Abschneiden nicht beschädigt werden, sollten die Schnitte in den Lücken zwischen den Wundauflagen **1** erfolgen. In der Figur ist eine solche Lücke mit dem Bezugszeichen **25** gekennzeichnet. Wenn die Lücken von außen nicht erkennbar sind (weil der Schlauch beispielsweise aus einem undurchsichtigen Material ohne Maschen besteht), ist es von Vorteil wie bei der Rolle **24** linienartige Indizierungen vorzusehen. Diese Indizierungen (in der Figur sind zwei Indizierungen **26a** und **26b** dargestellt) können dem Anwender anzeigen, wo geschnitten werden kann, ohne dass dabei die Gefahr besteht die Wundauflagen zu treffen. Obwohl in **Figur 6** jeweils nur eine Indizierung in Form einer durchgehenden Linie in einer Lücke dargestellt ist, können auch mehrere solcher Indizierungen in einer Lücke vorhanden sein.

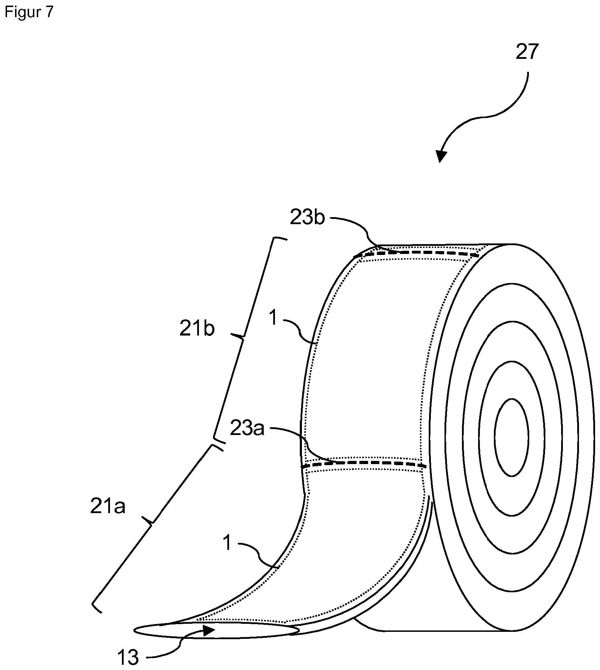

**Figur 7** zeigt eine Rolle **27** in einer dritten Ausführungsform der Erfindung. Die Rolle **27** umfasst eine Vielzahl von Verbänden nach **Figur 4**. Ansonsten ist die Rolle **27** wie die zuvor beschriebenen Rolle **22** ausgestaltet. Wie in der Figur angedeutet, schließen die Wundauflagen **1** lückenlos aneinander an und bilden dabei eine bahnartige Anordnung aus.

Dieses Konzept ermöglicht eine gute Anpassbarkeit des erfindungsgemäßen Verbandes an verschiedene Wundgrößen. Zur Versorgung einer kleineren Wunde kann zum Beispiel die Entnahme des Verbandes **21a** von der Rolle **27** genügen. Wenn die Wundoberfläche aber so groß ist, dass sie nicht mehr von einer einzigen Wundauflage 1 abgedeckt werden kann, so können von der Rolle **27** einfach zwei oder mehr Einzelverbände am Stück entnommen werden. Diese einfache Anpassbarkeit an verschiedene Wundgrößen ist bei den Rollen **22**, **24** aufgrund der Lücken zwischen den Wundauflagen nicht ohne weiteres gegeben.

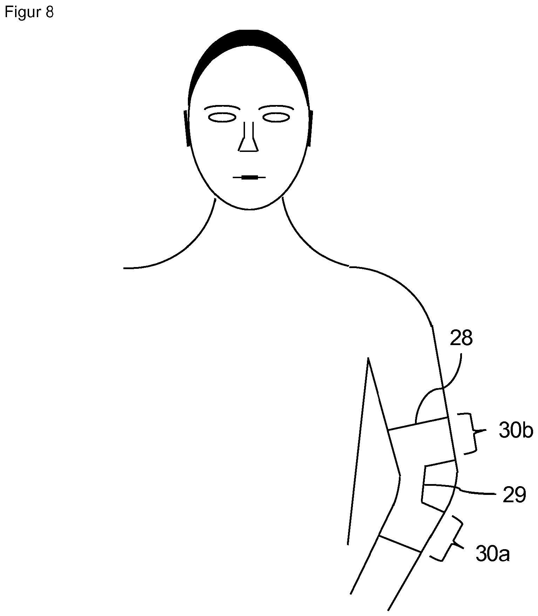

**Figur 8** zeigt einen Patienten mit einem Verband **28** gemäß einer vierten Ausführungsform der Erfindung. Der Verband **28** entspricht dem in den **Figuren 2a** **und** **2b** beschriebenen Verband **11**. Damit die Position der Wundauflage auch leicht bei Betrachtung der Außenseite des Verbandes ausgemacht werden kann, weist der Verband **28** zusätzlich noch eine Umrisszeichnung **29** auf. Die Umrisszeichnung **29** gibt exakt an, welcher Teil des Schlauches die Wundauflage überdeckt und erleichtert somit die Positionierung der Wundauflage über der Wunde. Eine solche Umrisszeichnung ist insbesondere dann vorteilhaft, wenn das Schlauchmaterial undurchsichtig ist und keine Maschen aufweist (also zum Beispiel aus einem dichten Gewebe oder Vlies besteht). Der Verband **28** wird in dem dargestellten Fall dazu verwendet, um eine Wunde am Ellbogen des Patienten zu behandeln. Durch die zentrale Anordnung der Wundauflage in dem Schlauch und die Schlauchüberstände **30a** und **30b** kann der Verband **28** die Wundauflage selbst am Ellbogen dauerhaft positioniert halten.

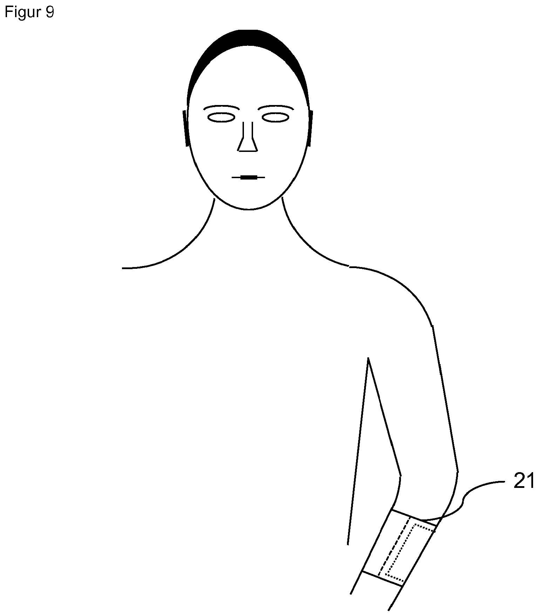

Schließlich zeigt **Figur 9** einen Patienten, der zur Behandlung einer Wunde am Unterarm einen Verband **21** (**Figur 4**) trägt. Da der Verband in diesem Fall kein Gelenk umfängt und daher weniger stark beansprucht wird als der Verband **28**, kann auf die zuvor erwähnten Schlauchüberstände verzichtet werden.

Der Durchmesser eines elastischen Schlauches zum Fixieren von Wundauflagen ist üblicherweise an den Durchmesser des Körperteils, welches von dem Schlauch aufgenommen werden soll, angepasst. Meistens ist der Durchmesser eines elastischen Schlauches zum Fixieren von Wundauflagen kleiner als der Durchmesser des Körperteils, welches von dem Schlauch aufgenommen werden soll. Entsprechend unterscheiden sich die Verbände aus **Figur 8** **und** **9** gegebenenfalls auch in ihren Schlauchdurchmessern, da der Oberarm in der Regel einen größeren Umfang als der Unterarm hat. Die Bereitstellung von Schläuchen zum Fixieren von Wundauflagen in unterschiedlichen Größen beziehungsweise Durchmessern ist im Stand der Technik bekannt und übliche Praxis.

## Patentansprüche

1. Verband (11, 17, 21, 28) zur Behandlung einer Wunde, umfassend
- eine absorbierende SAP-haltige Wundauflage (1), wobei die Wundauflage (1) eine der Wunde zugewandte erste Seite (2) und eine der Wunde abgewandte zweite Seite (3) aufweist,
- einen elastischen Schlauch (12) zum Fixieren der Wundauflage (1), wobei der Schlauch (12) ein erstes offenes Ende (13), ein zweites offenes Ende (14), eine der Wunde zugewandte Innenseite (15) und eine der Wunde abgewandte Außenseite (16) aufweist,
**dadurch gekennzeichnet, dass**
die zweite Seite (3) der Wundauflage (1) derart an der Innenseite (15) des Schlauches (12) unlösbar befestigt ist, dass die zweite Seite (3) der Wundauflage (1) vollständig von der Innenseite (15) des Schlauches (12) überdeckt ist.

2. Verband nach Anspruch 1, **dadurch gekennzeichnet, dass** die Wundauflage eine hydrophobe, im Wesentlichen flüssigkeitsundurchlässige und vorzugsweise atmungsaktive Schicht umfasst, so dass ein Flüssigkeitsübertritt von der Wundauflage in den Schlauch im Wesentlichen verhindert werden kann.

3. Verband (11) nach Anspruch 1 oder 2, **dadurch gekennzeichnet, dass** die Wundauflage (1)
- eine die erste Seite (2) der Wundauflage bildende Wundkontaktschicht (4),
- einen absorbierenden Kern (8) mit dem SAP und
- eine die zweite Seite (3) der Wundauflage bildende Rückschicht (5) umfasst.

4. Verband nach einem der vorangehenden Ansprüche, **dadurch gekennzeichnet, dass** das SAP ein Polyacrylat ist und vorzugsweise in Partikelform vorliegt.

5. Verband nach einem der vorangehenden Ansprüche, **dadurch gekennzeichnet, dass** die Wundauflage, insbesondere der absorbierende Kern der Wundauflage, eine Saugleistung von mindestens 50g/100cm², mindestens 100g/100cm² oder mindestens 140g/100cm², gemessen nach DIN EN 13726-1 (Kapitel 3.2), aufweist.

6. Verband nach einem der vorangehenden Ansprüche, **dadurch gekennzeichnet, dass** der Schlauch im Wesentlichen flüssigkeitsundurchlässig ausgestaltet ist.

7. Verband nach einem der vorangehenden Ansprüche, **dadurch gekennzeichnet, dass** der Schlauch hydrophob und vorzugsweise atmungsaktiv ist.

8. Verband nach einem der vorangehenden Ansprüche, **dadurch gekennzeichnet, dass** der Schlauch Bereiche unterschiedlicher Elastizität aufweist, wobei insbesondere ein die Wundauflage überdeckender Bereich des Schlauches eine geringere Elastizität als ein nicht die Wundauflage überdeckender Bereich des Schlauches aufweist.

9. Verband nach einem der vorangehenden Ansprüche, **dadurch gekennzeichnet, dass** der Schlauch nicht-adhäsiv ausgebildet ist.

10. Verband (17) nach einem der vorangehenden Ansprüche, **dadurch gekennzeichnet, dass** der Schlauch mindestens einen Silikonbund (18, 19) aufweist.

11. Verband (17) nach einem der vorangehenden Ansprüche, **dadurch gekennzeichnet, dass** der Schlauch eine Sollriss-Stelle (20) aufweist.

12. Verband nach einem der vorangehenden Ansprüche, **dadurch gekennzeichnet, dass** ein Teilbereich der zweiten Seite der Wundauflage oder die gesamte zweite Seite der Wundauflage an der Innenseite des Schlauches unlösbar befestigt ist.

13. Verband nach einem der vorangehenden Ansprüche, **dadurch gekennzeichnet, dass** ein die Wundauflage überdeckender Bereich des Schlauches an der Außenseite visuell erkennbar ist.

14. Verband (28) nach Anspruch 13, **dadurch gekennzeichnet, dass** der die Wundauflage überdeckende Bereich des Schlauches an der Außenseite durch eine Umrisszeichnung (29) und/oder durch eine farbige Markierung visuell erkennbar ist.

15. Verband nach einem der vorangehenden Ansprüche, **dadurch gekennzeichnet, dass** die Wundauflage elastisch ausgebildet ist.

16. Rolle (22, 24, 27) umfassend eine Vielzahl von Verbänden nach einem der vorangehenden Ansprüche, wobei die Verbände trennbar miteinander verbunden sind.

17. Anordnung von Verbänden zur Behandlung von Wunden an unterschiedlichen Körperstellen, umfassend
- einen oder mehrere erste Verbände nach einem der Ansprüche 1 bis 15, wobei die Wundauflage eines jeden ersten Verbandes an das jeweilige erste und zweite offene Ende angrenzt, und
- einen oder mehrere zweite Verbände nach einem der Ansprüche 1 bis 15, wobei die Wundauflage eines jeden zweiten Verbandes von dem jeweiligen ersten und zweiten offenen Ende beabstandet ist, und wobei der Abstand der Wundauflage eines jeden zweiten Verbandes von dem jeweiligen ersten und zweiten offenen Ende vorzugsweise jeweils mindestens etwa 2 cm, 4 cm oder 6 cm beträgt.

18. Verfahren zur Herstellung eines Verbandes für die Behandlung einer Wunde nach einem der Ansprüche 1 bis 15, umfassend die Schritte:
i. Bereitstellen einer SAP-haltigen Wundauflage, wobei die Wundauflage eine der Wunde zugewandte erste Seite und eine der Wunde abgewandte zweite Seite aufweist,
ii. Bereitstellen eines elastischen Schlauches zum Fixieren der Wundauflage, wobei der Schlauch ein erstes offenes Ende, ein zweites offenes Ende, eine der Wunde zugewandte Innenseite und eine der Wunde abgewandte Außenseite aufweist,
iii. Befestigen der zweiten Seite der Wundauflage an der Innenseite des Schlauches, so dass Wundauflage und Schlauch unlösbar miteinander verbunden sind und die zweite Seite der Wundauflage vollständig von der Innenseite des Schlauches überdeckt ist.

## Claims

1. Dressing (11, 17, 21, 28) for treating a wound, comprising
- an absorbent SAP-containing wound pad (1), wherein the wound pad (1) has a first side (2) facing the wound and a second side (3) facing away from the wound,
- an elastic sleeve (12) for holding the wound pad (1) in place, wherein the sleeve (12) has a first open end (13), a second open end (14), an inner side (15) facing the wound and an outer side (16) facing away from the wound,
**characterized in that**
the second side (3) of the wound pad (1) is fixed in an undetachable manner to the inner side (15) of the sleeve (12) such that the second side (3) of the wound pad (1) is completely covered by the inner side (15) of the sleeve (12).

2. Dressing according to Claim 1, **characterized in that** the wound pad includes a hydrophobic layer that is essentially impermeable to liquid and preferably breathable, such that liquid transfer from the wound pad to the sleeve can be essentially prevented.

3. Dressing (11) according to Claim 1 or 2, **characterized in that** the wound pad (1) includes
- a wound-contact layer (4) forming the first side (2) of the wound pad,
- an absorbent core (8) containing the SAP and
- a backing layer (5) forming the second side (3) of the wound pad.

4. Dressing according to any of the preceding claims, **characterized in that** the SAP is a polyacrylate and present preferably in particle form.

5. Dressing according to any of the preceding claims, **characterized in that** the wound pad, more particularly the absorbent core of the wound pad, has a suction capacity of at least 50 g/100 cm², at least 100 g/100 cm² or at least 140 g/100 cm², measured in accordance with DIN EN 13726-1 (section 3.2).

6. Dressing according to any of the preceding claims, **characterized in that** the sleeve is designed to be essentially impermeable to liquids.

7. Dressing according to any of the preceding claims, **characterized in that** the sleeve is hydrophobic and preferably breathable.

8. Dressing according to any of the preceding claims, **characterized in that** the sleeve has areas of varying elasticity, wherein more particularly an area of the sleeve covering the wound pad has lower elasticity than an area of the sleeve not covering the wound pad.

9. Dressing according to any of the preceding claims, **characterized in that** the sleeve is designed to be non-adhesive.

10. Dressing (17) according to any of the preceding claims, **characterized in that** the sleeve has at least one silicone collar (18, 19).

11. Dressing (17) according to any of the preceding claims, **characterized in that** the sleeve has a designated tear point (20).

12. Dressing according to any of the preceding claims, **characterized in that** a part-area of the second side of the wound pad or the entire second side of the wound pad is fixed in an undetachable manner to the inner side of the sleeve.

13. Dressing according to any of the preceding claims, **characterized in that** an area of the sleeve covering the wound pad is visually recognizable on the outside.

14. Dressing (28) according to Claim 13, **characterized in that** the area of the sleeve covering the wound pad is visually recognizable on the outside by a drawn outline (29) and/or by a coloured marking.

15. Dressing according to any of the preceding claims, **characterized in that** the wound pad is designed to be elastic.

16. Roll (22, 24, 27) comprising a plurality of dressings according to any of the preceding claims, wherein the dressings are connected to one another in a separable manner.

17. Arrangement of dressings for treating wounds in various parts of the body, comprising
- one or more first dressings according to any of Claims 1 to 15, wherein the wound pad of each first dressing abuts the first and second open end in each case, and
- one or more second dressings according to any of Claims 1 to 15, wherein the wound pad of each second dressing is spaced a distance apart from the first and second open end in each case, and wherein the distance of the wound pad of each second dressing from the first and second open end in each case is preferably in each case at least 2 cm, 4 cm or 6 cm.

18. Process for producing a dressing for treating a wound according to any of Claims 1 to 15, comprising the steps of:
i. providing an SAP-containing wound pad, wherein the wound pad has a first side facing the wound and a second side facing away from the wound,
ii. providing an elastic sleeve for holding the wound pad in place, wherein the sleeve has a first open end, a second open end, an inner side facing the wound and an outer side facing away from the wound,
iii. fixing the second side of the wound pad to the inner side of the sleeve, such that the wound pad and sleeve are connected to one another in an undetachable manner and the second side of the wound pad is completely covered by the inner side of the sleeve.

## Revendications

1. Bandage (11, 17, 21, 28) pour le traitement d'une plaie, comprenant :
- un pansement absorbant (1) contenant un SAP, le pansement (1) présentant un premier côté (2) tourné vers la plaie et un deuxième côté (3) détourné de la plaie,
- un tube élastique (12) pour fixer le pansement (1), le tube (12) présentant une première extrémité ouverte (13), une deuxième extrémité ouverte (14), un côté intérieur (15) tournée vers la plaie et un côté extérieur (16) détourné de la plaie,
**caractérisé en ce que**
le deuxième côté (3) du pansement (1) est fixé de manière inamovible au côté intérieur (15) du tube (12), de telle sorte que le deuxième côté (3) du pansement (1) est entièrement recouvert par le côté intérieur (15) du tube (12).

2. Bandage selon la revendication 1, **caractérisé en ce que** le pansement comprend une couche hydrophobe, essentiellement imperméable aux liquides et de préférence respirante, de telle sorte qu'un transfert de liquide du pansement dans le tube peut être essentiellement empêché.

3. Bandage (11) selon la revendication 1 ou 2, **caractérisé en ce que** le pansement (1) comprend :
- une couche de contact avec la plaie (4) formant le premier côté (2) du pansement,
- un noyau absorbant (8) comprenant le SAP et
- une couche arrière (5) formant le deuxième côté (3) du pansement.

4. Bandage selon l'une quelconque des revendications précédentes, **caractérisé en ce que** le SAP est un polyacrylate et se présente de préférence sous forme particulaire.

5. Bandage selon l'une quelconque des revendications précédentes, **caractérisé en ce que** le pansement, notamment le noyau absorbant du pansement, présente une capacité d'absorption d'au moins 50 g/100 cm², d'au moins 100 g/100 cm² ou d'au moins 140 g/100 cm², mesurée selon la norme DIN EN 13726-1 (chapitre 3.2).

6. Bandage selon l'une quelconque des revendications précédentes, **caractérisé en ce que** le tube est conçu de manière à être essentiellement imperméable aux liquides.

7. Bandage selon l'une quelconque des revendications précédentes, **caractérisé en ce que** le tube est hydrophobe et de préférence respirant.

8. Bandage selon l'une quelconque des revendications précédentes, **caractérisé en ce que** le tube présente des zones d'élasticité différente, une zone du tube recouvrant le pansement présentant notamment une élasticité plus faible qu'une zone du tube ne recouvrant pas le pansement.

9. Bandage selon l'une quelconque des revendications précédentes, **caractérisé en ce que** le tube est configuré sous forme non adhésive.

10. Bandage (17) selon l'une quelconque des revendications précédentes, **caractérisé en ce que** le tube présente au moins un rebord en silicone (18, 19).

11. Bandage (17) selon l'une quelconque des revendications précédentes, **caractérisé en ce que** le tube présente un point de rupture de consigne (20).

12. Bandage selon l'une quelconque des revendications précédentes, **caractérisé en ce qu'**une zone partielle du deuxième côté du pansement ou la totalité du deuxième côté du pansement est fixée de manière inamovible au côté intérieur du tube.

13. Bandage selon l'une quelconque des revendications précédentes, **caractérisé en ce qu'**une zone du tube recouvrant le pansement est visuellement identifiable sur le côté extérieur.

14. Bandage (28) selon la revendication 13, **caractérisé en ce que** la zone du tube recouvrant le pansement est visuellement identifiable sur le côté extérieur par un dessin de contour (29) et/ou par un marquage coloré.

15. Bandage selon l'une quelconque des revendications précédentes, **caractérisé en ce que** le pansement est configuré sous forme élastique.

16. Rouleau (22, 24, 27) comprenant une pluralité de bandages selon l'une quelconque des revendications précédentes, les bandages étant reliés entre eux de manière séparable.

17. Agencement de bandages pour le traitement de plaies à différents endroits du corps, comprenant :
- un ou plusieurs premiers bandages selon l'une quelconque des revendications 1 à 15, le pansement de chaque premier bandage étant adjacent aux première et deuxième extrémités ouvertes respectives, et
- un ou plusieurs deuxièmes bandages selon l'une quelconque des revendications 1 à 15, le pansement de chaque deuxième bandage étant espacé des première et deuxième extrémités ouvertes respectives, et l'espacement du pansement de chaque deuxième bandage par rapport aux première et deuxième extrémités ouvertes respectives étant de préférence d'au moins environ 2 cm, 4 cm ou 6 cm.

18. Procédé de fabrication d'un bandage pour le traitement d'une plaie selon l'une quelconque des revendications 1 à 15, comprenant les étapes suivantes :
i. la fourniture d'un pansement contenant un SAP, le pansement présentant un premier côté tourné vers la plaie et un deuxième côté détourné de la plaie,
ii. la fourniture d'un tube élastique pour fixer le pansement, le tube présentant une première extrémité ouverte, une deuxième extrémité ouverte, un côté intérieur tourné vers la plaie et un côté extérieur détourné de la plaie,
iii. la fixation du deuxième côté du pansement au côté intérieur du tube, de telle sorte que le pansement et le tube sont reliés de manière inamovible l'un à l'autre et le deuxième côté du pansement est entièrement recouvert par le côté intérieur du tube.
